# EUROPEAN PATENT SPECIFICATION

(11) **EP 3 930 853 B1**
(45) Date of publication and mention of the grant of the patent: **17.06.2026**
(21) Application number: 20709341.0
(22) Date of filing: 27.02.2020
(51) Int. Cl.: A61Q 5/12, A61Q 5/06, A61K 8/41, A61K 8/34, A61K 8/85, A61K 8/26, A61K 8/73, A61K 8/02

(54) **COSMETIC FORMULATION, PROCESS FOR ITS PRODUCTION AND METHOD OF USE OF THE SAME AS COSMETIC HAIR TREATMENT ALTERNATIVE TO SILICONES**
KOSMETISCHE ZUSAMMENSETZUNG, VERFAHREN ZU DEREN HERSTELLUNG UND METHODE ZUR KOSMETISCHEN BEHANDLUNG VON HAAREN ALS ALTERNATIVE ZU SILIKONEN
COMPOSITION COSMÉTIQUE, PROCÉDÉ DE PRÉPARATION ET MÉTHODE D'UTILISATION EN TANT QUE TRAITEMENT DES CHEVEUX ALTERNATIF AUX SILICONES

(30) Priority: 01.03.2019 IT 201900003053
(43) Date of publication of application: 05.01.2022
(73) Proprietor: Pettenon Cosmetics S.p.A. Società Benefit, 35018 San Martino di Lupari (PD) (IT)
(72) Inventor: BARBUZZI, Elena Maria Gabriella, 35018 San Martino di Lupari (PD) (IT); MARTINI, Maria Luisa, 35018 San Martino di Lupari (PD) (IT); PEGORIN, Gianni, 35018 San Martino di Lupari (PD) (IT)
(74) Representative: Rocchetto, Elena
(86) International application number: PCT/IB2020/051671
(87) International publication number: WO 2020/178674

(56) References cited:
- WO-A1-2013/074655
- US-A1- 2018 092 825
- US-A1- 2019 029 948
- "Bounce Up Treatment & Pack", GNPD, MINTEL, 1 August 2016 (2016-08-01), XP002774094
- DATABASE GNPD [online] MINTEL; 5 October 2017 (2017-10-05), ANONYMOUS: "Nourishing Body Smoother", XP055633653, retrieved from www.gnpd.com Database accession no. 5123329
- DATABASE GNPD [online] MINTEL; 1 March 2018 (2018-03-01), ANONYMOUS: "Modeling Pomade", XP055633612, retrieved from www.gnpd.com Database accession no. 5483503
- DATABASE GNPD [online] MINTEL; 31 October 2018 (2018-10-31), ANONYMOUS: "Rebalancing Conditioner", XP055633718, retrieved from www.gnpd.com Database accession no. 6083581
- DATABASE GNPD [online] MINTEL; 31 January 2019 (2019-01-31), ANONYMOUS: "Rapid Reviver Deep Conditioner", XP055633606, retrieved from www.gnpd.com Database accession no. 6303911

## Description

### Technical field of the invention

The present patent concerns the field of cosmetic formulations for hair care in general, and more specifically it concerns a new cosmetic formulation, the process for its production and the method of use of the cosmetic formulation for hair care in a cosmetic treatment which is an alternative to the use of silicones.

### State of the art

The cosmetic formulations for shampoos are generally structured with very effective cleansing surfactants, typically anionic surfactants, and in themselves they are not particularly beneficial for the hair in terms of conditioning effect or styling. In fact, the simple formulations for shampoos which have not been supplemented with specific conditioning agents or hair styling aids tend to leave the hair in conditions which are not satisfying from the cosmetic point of view, in terms of both hair manageability and hair stylability. The hair tends to be stiff, opaque and dry to the touch, a condition which is often defined as "creak", in many cases is difficult to comb, both when wet and when dry, typically cannot be easily brushed and tends to have a reduced capacity to hold its style.

This has led to the use of products containing specific conditioning agents and/or agents suited to facilitate hair styling. These products are generally applied separately, after washing and rinsing the hair, for example in the form of hair conditioners or styling mousses, etc.

The formulations of conventional hair conditioners, though ensuring considerable improvements, for example in terms of easy combing when the hair is both wet and dry and of smoothness, do not facilitate hair styling in themselves, for example do not make the hair smooth and straight, especially when it is thick and rough hair. In fact, traditional hair conditioners tend to have a negative effect on many of the characteristics which are associated with the body of the hair: the use of silicones in high concentrations can have a considerable impact on the feeling of cleanliness. The hair seems to be greasy and heavy, with a negative feeling to the touch already on the second day after use, to the extent that the user feels the need to wash his/her hair more frequently. Another common negative effect of traditional hair conditioners is represented by the so-called "build up", meaning the accumulation of the conditioning agents on the surface of the hair after repeated uses, which causes the hair to become opaque and heavy.

One of the most common methods employed to improve the characteristics of the hair and facilitate hair styling was the use of setting agents, for example polymers with high molecular weight. The problem related to the use of setting agents lies in that the latter tend to have a negative effect on the results of conditioning, such as the capacity to produce a feeling of cleanliness with both wet and dry hair. On the contrary, these agents make the hair feel sticky. The database Mintel discloses several hair conditioning compositions that are free of silicone (see for example entries 6083581, 5483503, 4191539).

The conventional polymers used for hair styling are typically soluble in water. This means that when they are incorporated in a hair conditioner that is successively rinsed off they tend to be washed away together with the hair conditioner, to a more or less considerable extent. Consequently, most hair styling products are applied to the hair after the hair conditioner and then are left on the hair.

Various types of hair styling equipment and tools are known which, however, as shown by the available literature on the subject and experienced by consumers, can cause permanent damage to the hair, since high temperatures modify its chemical structure.

The problem addressed by the present invention concerns the production of compositions for hair conditioners to be rinsed off which are expected to offer advantages in terms of hair styling, and in particular to smooth the hair, producing the same effect which is obtained through the use of hair straighteners but without any negative impact on the conditioning properties of the hair conditioner, as it happens, instead, especially with the use of silicones which tend to make the hair heavy and greasy to the touch, with a tendency to easily get dirty.

The effects and characteristics that the present invention intends to achieve are, in particular, the following: silkiness, hair feeling smooth to the touch, hair that is brighter, more elastic, easier to comb (for example, easier to straighten) and to manage (for example, hair that holds its styling without feeling stiff or giving other negative feelings to the touch). These characteristics inherent to smooth hair are particularly appealing for people having thick, long, heavy hair which tends to easily get curly and to be difficult to manage in terms of both hair styling and volume.

The inventor has discovered that, with the pH levels commonly used in cosmesis, meaning levels which are generally included between 3.5 and 7, if a small quantity of tiny mineral particles with zeta potential (electrokinetic potential) included between -19 mV and -41 mV and particle size included between 10 and 200 µm is incorporated in a product, these mineral particles are deposited on the hair surface to neutralize negative charges, mainly concentrating towards the hair ends, which normally are the most damaged parts.

The product which is the subject of the present patent has a high capacity to absorb water, which stabilizes emulsions, helps improve the sensory effect of the emulsions themselves and reduces the negative feeling caused by the emulsions of the known type when applied to the hair, which consequently is oily and greasy to the touch.

The inventor has also discovered that the combined introduction of crosslinked polymerized starches, chemically modified and able to act as rheology modifiers, such as xanthan gum, carrageenan, guar gum, pectins, increases the conditioning effects of the product on dry hair.

Furthermore, the new formulation comprises also at least one non-ionic natural polymer with conditioning effect, with a molecular weight included between 150 and 3000 g/Mol and scarce solubility in water, which is distributed over the entire carrier, made up of a combination of solid particles and a modified starch polymer.

This combination makes it possible to obtain exceptional temporary effects for the hair in terms of smoothing effect: hair that is easy to disentangle when both wet and dry, hydrated, bright.

The modified starches used in this formulation are easy to disperse in water and are used for their capacity to act as rheological stabilizers of emulsions, to which they confer compactness, pleasantness to the touch and conditioning properties.

### Detailed description of the invention

The present invention concerns a water-based formulation for hair conditioners, comprising:
(i) at least one cationic surfactant;
(ii) at least one fatty alcohol;
(iii) a determined quantity of coated particles, in turn comprising: (a) inorganic solid particles (b) at least one chemically modified polymerized starch;
(iv) at least one non-ionic polymer, characterized in that:
   - said starch polymer is chemically modified and is a native starch of plant origin, such as corn starch tapioca starch, wheat starch, potato starch,
   - said non-ionic polymer is lauryl/myristyl polyricinoleate (and) glycerin and has a molecular weight included between 150 and 3000 g/Mol,
   - said at least one cationic surfactant is selected among Behentrimonium Chloride, Distearyldimonium Chloride, Cetrimonium Chloride, Dicetyldimonium Chloride;
   - said fatty alcohol is selected among Mirystyl Alcohol, Cetyl Alcohol, Cetearyl Alcohol, Stearyl Alcohol, Behenyl Alcohol, and more preferably is Cetearyl alcohol;
   - said inorganic solid particles are selected among kaolin, clays, hydroxyapatites, zinc oxides and titanium oxides, zeolites, bentonites, peloids, said inorganic solid particles having dimensions included between 10 and 200 /lm;
and wherein the formulation also comprises at least one fat obtained from plant seeds, wherein said at least one fat is selected among shea butter or butyrospermum parkii butter, cocoa butter, peanut butter, avocado butter, mango butter.

More specifically, said coated particles are present in a quantity included between 0,01 and 10 % in weight.

The present invention provides hair care products with properties that improve the appearance of the hair or enhances said aesthetic properties. These properties can make the hair smooth and silky to the touch as well as straight, and prevent it from becoming greasy and oily.

The inorganic particles have mineral/natural origin and are constituted by small silicate particles and several macro minerals.

One of the objects of the present invention is to make the product pleasant to the touch; the product is fluid, creamy, non-sticky and/or non-oily.

For example, the benefit provided by the chemically modified starches used in this formulation can be observed when the product is applied to the hair and rubbed so as to form a thin film on its surface; in fact, these starches make emulsions "shear thinning" with a rapid recovery of the structure once there is no more mechanical stress: the deposit of the active agents on the hair takes place through a thin emulsion, homogeneous and pleasant to the touch.

The starches used in the present invention are chemically modified and are native starches of plant origin, such as corn starch, tapioca starch, wheat starch, potato starch.

In the embodiment that is the subject of this experiment said starch derives from wheat.

The processes which are suitable for the modification of the starch comprise the following, without however being limited to them: etherification, esterification and degradation through the action of acids, oxidants and enzymes. The physical modifications can comprise thermal treatments, grinding, crushing and dextrinization (thermal and acid treatment) and other similar processes.

The new cosmetic formulation can be in the form of a hair mask.

The possible compositions are illustrated here below:

### Example no. 1

| ***CAS N.*** | ***INCI US Name*** | ***INCI EU Name*** | ***% in weight*** | ***Function*** |
|---|---|---|---|---|
| 7732-18-5 | Water | Aqua | 75 < x ≤ 100 | Solvent |
| 67762-27-0 | Cetearyl Alcohol | Cetearyl Alcohol | 5 < x ≤ 10 | Emulsion stabilizer |
| 8005-44-5 | | | | |
| 90622-77-8 | Cocamide MEA | Cocamide MEA | 1 < x ≤ 5 | Thickening agent |
| 68140-00-1 | | | | |
| 1332-58-7 | Kaolin | Kaolin | 0.1 < x ≤ 1 | Bleaching agent |
| 113894-92-1 | Hydroxypropyl Starch Phosphate | Hydroxypropyl Starch Phosphate | 0.1 < x ≤ 1 | Rheological additive |
| 122-99-6 | Phenoxyethanol | Phenoxyethanol | 0.1 < x ≤1 | Preservative agent |
| | Lauryl/Myristyl Polyricinoleate (and) Glycerin | Lauryl/Myristyl Polyricinoleate (and) Glycerin | 0.1< x ≤ 1 | |
| 194043-92-0 | Butyrospermum Parkii (Shea) Butter | Butyrospermum Parkii Butter | 0.1< x ≤ 1 | Organic additive |
| 17301-53-0 | Behentrimonium chloride | Behentrimonium chloride | 0.1< x ≤ 1 | Conditioning agent |
| 70445-33-9 | Ethylhexylglycerin | Ethylhexylglycerin | 0.0 ≤ x ≤ 0.1 | Solvent |
| 77 92 9 | Citric Acid | Citric Acid | 0.0 ≤ x ≤ 0.1 | pH regulator |
| 5949-29-1 | | | | |

### Example no. 2

| ***CAS N.*** | ***INCI US Name*** | ***INCI EU Name*** | ***% in weight*** | ***Function*** |
|---|---|---|---|---|
| 7732-18-5 | Water | Aqua | 75 < x ≤ 100 | Solvent |
| 67762-27-0 | Cetearyl Alcohol | Cetearyl Alcohol | 5 < x ≤ 10 | Emulsion stabilizer |
| 8005-44-5 | | | | |
| 112-02-7 | Cetrimonium Chloride | Cetrimonium Chloride | 1 < x ≤ 5 | Hair conditioning agent |
| | Lauryl/Myristyl Polyricinoleate | Lauryl/Myristyl Polyricinoleate (and) | 0,1< x ≤ 1 | |
| | (and) Glycerin | Glycerin | | |
| 149-32-6 | Erythritol | Erythritol | **0,1< x ≤ 1** | **Conditioning agent** Wetting agent Hydrating agent |
| 113894-92-1 | Hydroxypropyl Starch Phosphate | Hydroxypropyl Starch Phosphate | 0,1 < x ≤ 1 | Rheological additive |
| 122-99-6 | Phenoxyethanol | Phenoxyethanol | 0,1 < x ≤ 1 | Preservative agent |
| 1332-58-7 | Kaolin | Kaolin | 0,1 < x ≤ 1 | Bleaching agent |
| | Titanium Dioxide | Titanium Dioxide | 0,1 < x ≤ 1 | Bleaching agent |
| | Fragrance | Parfum | 0,1 < x ≤ 1 | Fragrance |
| 194043-92-0 | Butyrospermum Parkii (Shea) Butter | Butyrospermum Parkii Butter | 0,1< x ≤ 1 | Organic additive |
| 70445-33-9 | Ethylhexylglycerin | Ethylhexylglycerin | 0,0 ≤ x ≤ 0,1 | Solvent |
| 77929 | Citric Acid | Citric Acid | 0,0 ≤ x ≤ 0,1 | pH regulator |
| 5949-29-1 | | | | |
| 67-63-0 | Isopropyl Alcohol | Isopropyl Alcohol | 0,0 ≤ x ≤ 0,1 | Solvent |

### Example no. 3

| ***CAS N.*** | ***INCI US Name*** | ***INCI EU Name*** | ***% in weight*** | ***Function*** |
|---|---|---|---|---|
| 7732-18-5 | Water | Aqua | 75 < x ≤ 100 | Solvent |
| 67762-27-0 8005-44-5 | Cetearyl Alcohol | Cetearyl Alcohol | 5 < x ≤ 10 | Emulsion stabilizer |
| 112-02-7 | Cetrimonium Chloride | Cetrimonium Chloride | 1 < x ≤ 5 | Hair conditioning agent |
| 1332-58-7 | Kaolin | Kaolin | 0,1 < x ≤ 1 | Bleaching agent |
| 113894-92-1 | Hydroxypropyl Starch Phosphate | Hydroxypropyl Starch Phosphate | 0,1 < x ≤ 1 | Rheological additive |
| 122-99-6 | Phenoxyethanol | Phenoxyethanol | 0,1 < x ≤ 1 | Preservative agent |
| | Lauryl/Myristyl Polyricinoleate (and) Glycerin | Lauryl/Myristyl Polyricinoleate (and) Glycerin | 0,1< x ≤ 1 | Hair conditioning agent |
| 194043-92-0 | Butyrospermum Parkii (Shea) Butter | Butyrospermum Parkii Butter | 0,1< x ≤ 1 | Organic additive |
| 70445-33-9 | Ethylhexylglycerin | Ethylhexylglycerin | 0,0 ≤ x ≤ 0,1 | Solvent |
| 77929 | Citric Acid | Citric Acid | 0,0 ≤ x ≤ 0,1 | pH regulator |
| 5949-29-1 | | | | |
| 67-63-0 | Isopropyl Alcohol | Isopropyl Alcohol | 0,0 ≤ x ≤ 0,1 | Solvent |
| 112-69-6 | Dimethyl Palmitamine | Dimethyl Palmitamine | 0,0 ≤ x ≤ 0,1 | Emulsifying agent |

### Structure of the formula

In greater detail, the composition comprises:
i) at least one cationic surfactants selected among the following :
   ∘ Behentrimonium Chloride
   ∘ Distearyldimonium Chloride
   ∘ Cetrimonium Chloride
   ∘ Dicetyldimonium Chloride
ii) fatty alcohols, of plant, animal or synthetic origin, wherein the fatty alkyl alcohols are the following:
   ∘ Myristyl Alcohol
   ∘ Cetyl Alcohol
   ∘ Cetearyl Alcohol
   ∘ Stearyl Alcohol
   ∘ Behenyl Alcohol
iii) at least one starch-based chemically modified polymer; inorganic particles selected among kaolin, clays, hydroxyapatites, zinc oxides and titanium oxides, zeolites, bentonites, peloids said inorganic solid particles having dimensions included between 10 and 200 µm; said starch is a native starch with plant origin (maize, tapioca, wheat, potato);
iv) at least one non-ionic polymer, serving as a hair conditioning agent, with molecular weight included between 150 and 3000 g/Mol, wherein the non-ionic polymer is lauryl/myristyl polyricinoleate (and) glycerin;
v) at least one fat or fatty oil obtained from the seeds of a plant, wherein said fat is selected among shea butter or butyrospermum parkii butter, cocoa butter, peanut butter, avocado butter, mango butter.

### Production process

The production process comprises the following steps:
- introducing demineralized water in a container and heating the latter to 75 - 85°C;
- adding a pH regulator, for example citric acid, lactic acid, glycolic acid, followed by the addition, to be carried out slowly, of the modified starch and simultaneous mixing with a mechanical mixer;
- homogenizing the solution in a turbine for 1 minute;
- obtaining a uniform solution, continuing to mix and successively adding a non-ionic polymer, such as lauryl/myristyl polyricinoleate, and a cationic surfactant such as, for example, cetrimonium chloride;
- mixing again and adding a cationic surfactant, for example behentrimonium chloride, a fat, for example shea butter (butyrospermum parkii butter) and a fatty alcohol, for example cetearyl alcohol;
- checking the temperature, which must always be included in the range of values between 60 and 90 °C;
- if this is not the case, heating again until reaching a temperature included in the range of values between 60 and 90 °C;
- waiting for more than 5 minutes;
- leaving the solution to cool and, once a temperature value included between 65 and 85 °C, and more preferably between 50 and 80 °C, adding inorganic particles previously mixed with cold water, successively adding cold water;
- leaving the solution to cool;
- once a temperature value included between 30 and 55 °C and preferably between 35 and 50 °C has been reached, adding a preservative agent, also checking the pH, which must be included in the range of values between 3.0 and 5.0, and preferably between 3.0 and 4.0.

Therefore, with reference to the above description, the following claims are expressed.

## Claims

1. Water-based formulation, comprising:
(i) at least one cationic surfactant;
(ii) at least one fatty alcohol;
(iii) a determined quantity of coated particles, in turn comprising: (a) inorganic solid particles, (b) at least one modified starch polymer;
(iv) at least one non-ionic polymer,
**characterized in that**:
- said starch polymer is chemically modified and is a native starch of plant origin, such as corn starch, tapioca starch, wheat starch, potato starch,
- said non-ionic polymer is lauryl/myristyl polyricinoleate (and) glycerin and has a molecular weight included between 150 and 3000 g/Mol,
- said at least one cationic surfactant is selected among Behentrimonium Chloride, Distearyldimonium Chloride, Cetrimonium Chloride, Dicetyldimonium Chloride;
- said fatty alcohol is selected among Myristyl Alcohol, Cetyl Alcohol, Cetearyl Alcohol, Stearyl Alcohol, Behenyl Alcohol, and more preferably is Cetearyl alcohol;
- said inorganic solid particles are selected among kaolin, clays, hydroxyapatites, zinc oxides and titanium oxides, zeolites, bentonites, peloids, said inorganic solid particles having dimensions included between 10 and 200 µm;
and wherein the formulation also comprises at least one fat obtained from plant seeds, wherein said at least one fat is selected among shea butter or butyrospermum parkii butter, cocoa butter, peanut butter, avocado butter, mango butter.

2. Formulation according to claim 1, **characterized in that** said at least one cationic surfactant is present therein in a quantity included between 0.01 and 10% in weight, preferably higher than 5%.

3. Formulation according to claim 1, **characterized in that** said fatty alcohol is present therein in a quantity included between 0.01 and 15% in weight, preferably higher than 12%.

4. Formulation according to claim 1, **characterized in that** said coated particles are present therein in a quantity included between said 0.01 and 10% in weight, preferably higher than 3%.

5. Hair care product or mask, comprising the formulation according to one or more of the preceding claims.

6. Cosmetic hair treatment method, **characterized in that** it comprises the application of the hair mask according to claim 5 to the hair, the rubbing of the same product on the hair so as to form a thin film on its surface and the successive rinsing of the product off the hair.

7. Process for obtaining a formulation according to one or more of the claims from 1 to 4, **characterized in that** it comprises the following steps:
- preparation of a cream under controlled temperature in the range included between 60 and 90°C, wherein said cream is obtained by adding to water, in succession, at least one modified starch, at least one non-ionic polymer, a cationic surfactant, a fat, a fatty alcohol;
- cooling in the temperature range included between 65 and 85°C, and more preferably between 50 and 80°C;
- addition of inorganic particles mixed with cold water and successive addition of cold water;
- continuation of the cooling process to reach a temperature included between 30 and 55°C, preferably between 35 and 50 °C;
- addition of a preservative and pH control intended to keep the pH value within the range between 3.0 and 5.0, and preferably between 3.0 and 4.0.

## Patentansprüche

1. Formulierung auf Wasserbasis, umfassend:
(i) mindestens ein kationisches Tensid;
(ii) mindestens einen Fettalkohol;
(iii) eine bestimmte Menge beschichteter Partikel, die wiederum Folgendes umfassen: (a) anorganische Feststoffpartikel, (b) mindestens ein modifiziertes Stärkepolymer;
(iv) mindestens ein nichtionisches Polymer,
**dadurch gekennzeichnet, dass**:
- das besagte Stärkepolymer chemisch modifiziert ist und eine native Stärke pflanzlichen Ursprungs ist, wie Maisstärke, Tapiokastärke, Weizenstärke, Kartoffelstärke,
- das besagte nichtionische Polymer Lauryl/Myristyl Polyricinoleat (und) Glycerin ist und ein Molekulargewicht zwischen 150 und 3000 g/Mol hat,
- das besagte mindestens eine kationische Tensid ausgewählt ist aus Behentrimoniumchlorid, Distearyldimoniumchlorid, Cetrimoniumchlorid, Dicetyldimoniumchlorid;
- der besagte Fettalkohol ausgewählt ist aus Myristylalkohol, Cetylalkohol, Cetearylalkohol, Stearylalkohol, Behenylalkohol und besonders bevorzugt Cetearylalkohol ist;
- die besagten anorganischen Feststoffpartikel ausgewählt sind aus Kaolin, Tonen, Hydroxylapatiten, Zinkoxiden und Titanoxiden, Zeolithen, Bentoniten, Peloiden, wobei die besagten anorganischen Feststoffpartikel eine Größe zwischen 10 und 200 µm haben;
und wobei die Formulierung ferner mindestens ein Fett umfasst, das aus Pflanzensamen erhalten wird, wobei das besagte mindestens eine Fett aus Sheabutter oder Butyrospermum Parkii Butter, Kakaobutter, Erdnussbutter, Avocadobutter, Mangobutter ausgewählt ist.

2. Formulierung nach Patentanspruch 1, **dadurch gekennzeichnet, dass** das besagte mindestens eine kationische Tensid darin in einer Menge zwischen 0,01 und 10 Gew.-%, vorzugsweise höher als 5 %, vorhanden ist.

3. Formulierung nach Patentanspruch 1, **dadurch gekennzeichnet, dass** der besagte Fettalkohol darin in einer Menge zwischen 0,01 und 15 Gew.-%, vorzugsweise höher als 12 %, enthalten ist.

4. Formulierung nach Patentanspruch 1, **dadurch gekennzeichnet, dass** die besagten beschichteten Partikel darin in einer Menge zwischen dem besagten 0,01 und 10 Gew.-%, vorzugsweise höher als 3 %, vorhanden sind.

5. Haarpflegeprodukt oder -maske, umfassend die Formulierung nach einem oder mehreren der vorhergehenden Patentansprüche.

6. Kosmetisches Haarbehandlungsverfahren, **dadurch gekennzeichnet, dass** es die folgenden Schritte umfasst: das Auftragen der Haarmaske nach Patentanspruch 5 auf das Haar, das Einreiben desselben Produkts in das Haar, um eine dünne Schicht auf dessen Oberfläche zu bilden, und das nachfolgende Ausspülen des Produkts aus dem Haar.

7. Verfahren zum Erhalten einer Formulierung nach einem oder mehreren der Patentansprüche 1 bis 4, **dadurch gekennzeichnet, dass** es die folgenden Schritte umfasst:
- Herstellung einer Creme bei kontrollierter Temperatur im Bereich zwischen 60 und 90 °C, wobei die besagte Creme erhalten wird, indem nacheinander mindestens eine modifizierte Stärke, mindestens ein nichtionisches Polymer, ein kationisches Tensid, ein Fett, ein Fettalkohol zu Wasser zugegeben werden;
- Kühlung im Temperaturbereich zwischen 65 und 85 °C, und besonders bevorzugt zwischen 50 und 80 °C;
- Zugabe von mit Kaltwasser vermischten anorganischen Partikeln und nachfolgende Zugabe von Kaltwasser;
- Fortsetzung des Kühlverfahrens bis zum Erreichen einer Temperatur zwischen 30 und 55 °C, vorzugsweise zwischen 35 und 50 °C;
- Zugabe eines Konservierungsmittels und pH-Kontrolle, um den pH-Wert im Bereich zwischen 3,0 und 5,0, vorzugsweise zwischen 3,0 und 4,0, zu halten.

## Revendications

1. Formulation à base d'eau, comprenant :
(i) au moins un tensioactif cationique ;
(ii) au moins un alcool gras ;
(iii) une quantité déterminée de particules enrobées, comprenant à leur tour : (a) des particules solides inorganiques, (b) au moins un polymère d'amidon modifié ;
(iv) au moins un polymère non ionique,
**caractérisée en ce que** :
- ledit polymère d'amidon est chimiquement modifié et est un amidon natif d'origine végétale, tel que l'amidon de maïs, l'amidon de tapioca, l'amidon de blé, l'amidon de pomme de terre,
- ledit polymère non ionique est le polyricinoléate de lauryle/myristyle (et) la glycérine et présente un poids moléculaire compris entre 150 et 3 000 g/mol,
- - ledit au moins un tensioactif cationique est choisi parmi le chlorure de béhentrimonium, le chlorure de distéaryldimonium, le chlorure de cétrimonium, le chlorure de dicétyldimonium ;
- ledit alcool gras est choisi parmi l'alcool myristylique, l'alcool cétylique, l'alcool cétéarylique, l'alcool stéarylique, l'alcool béhénylique, et est de préférence l'alcool cétéarylique ;
- lesdites particules solides inorganiques sont choisies parmi le kaolin, les argiles, les hydroxyapatites, les oxydes de zinc et les oxydes de titane, les zéolites, les bentonites, les péloïdes, lesdites particules solides inorganiques ayant des dimensions comprises entre 10 et 200 µm ;
et où la formulation comprend également au moins une graisse obtenue à partir de graines végétales, où ladite au moins une graisse est choisie parmi le beurre de karité ou beurre de Butyrospermum parkii, le beurre de cacao, le beurre d'arachide, le beurre d'avocat et le beurre de mangue.

2. Formulation selon la revendication 1, **caractérisée en ce que** ledit au moins un tensioactif cationique y est présent en une quantité comprise entre 0,01 et 10 % en poids, de préférence supérieure à 5 %.

3. Formulation selon la revendication 1, **caractérisée en ce que** ledit alcool gras y est présent en une quantité comprise entre 0,01 et 15 % en poids, de préférence supérieure à 12 %.

4. Formulation selon la revendication 1, **caractérisée en ce que** lesdites particules enrobées y sont présentes en une quantité comprise entre lesdits 0,01 et 10 % en poids, de préférence supérieure à 3 %.

5. Produit de soin capillaire ou masque, comprenant la formulation selon l'une ou plusieurs des revendications précédentes.

6. Procédé de traitement cosmétique des cheveux, **caractérisé en ce qu'**il comprend l'application du masque capillaire selon la revendication 5 sur les cheveux, le frottement du même produit sur les cheveux de manière à former un film mince sur leur surface et le rinçage successif du produit des cheveux.

7. Procédé pour l'obtention d'une formulation selon l'une ou plusieurs des revendications 1 à 4, **caractérisé en ce qu'**il comprend les étapes suivantes :
- la préparation d'une crème à une température contrôlée comprise entre 60 et 90 °C, où ladite crème est obtenue en ajoutant à de l'eau, successivement, au moins un amidon modifié, au moins un polymère non ionique, un tensioactif cationique, une graisse, un alcool gras ;
- le refroidissement dans la plage de températures comprise entre 65 et 85 °C, et de préférence entre 50 et 80 °C ;
- l'ajout de particules inorganiques mélangées à de l'eau froide et l'ajout successif d'eau froide ;
- la continuation du processus de refroidissement jusqu'à atteindre une température comprise entre 30 et 55 °C, de préférence entre 35 et 50 °C ;
- l'ajout d'un conservateur et d'un régulateur de pH destiné à maintenir le pH dans une plage comprise entre 3,0 et 5,0, et de préférence entre 3,0 et 4,0.
